(19) Europäisches Patentamt
European Patent Office
Office européen des brevets

(11) Veröffentlichungsnummer: **0 054 228**
**A1**

# (12) EUROPÄISCHE PATENTANMELDUNG

(21) Anmeldenummer: **81110103.9**

(22) Anmeldetag: **03.12.81**

(51) Int. Cl.$^3$: **C 07 C 93/14**

(30) Priorität: **11.12.80 DE 3046607**

(43) Veröffentlichungstag der Anmeldung:
**23.06.82 Patentblatt 82/25**

(84) Benannte Vertragsstaaten:
**CH DE FR GB IT LI**

(71) Anmelder: **HOECHST AKTIENGESELLSCHAFT**
**Postfach 80 03 20**
**D-6230 Frankfurt/Main 80(DE)**

(72) Erfinder: **Gengnagel, Kurt, Dr.**
**Rossertstrasse 88**
**D-6239 Kriftel(DE)**

(72) Erfinder: **Papenfuhs, Theodor, Dr.**
**Heinrich-Bleicher-Strasse 40**
**D-6000 Frankfurt am Main 50(DE)**

(54) **Verfahren zur Herstellung von 1-Amino-2- oder -4-nitro-alkoxybenzolen.**

(57) 1-Amino-2-nitro-alkoxybenzole und 1-Amino-4-nitro-alkoxybenzole mit einer oder zwei Alkoxygruppen lassen sich aus den entsprechenden 1-Acetylamino-Verbindungen durch Hydrolyse mittels wasserfreiem oder praktisch wasserfreiem Alkanol von 1 bis 5 C-Atomen unter Verwendung von geringen Mengen an einem Alkalihydroxid rein herstellen, ohne daß hierbei Abwasser entsteht. Die alkalische Alkoholyse wird bevorzugt bei Rückflußtemperatur des Reaktionsmediums durchgeführt. Eine Umkristallisation des Endproduktes ist nicht mehr erforderlich. Das alkoholische Reaktionsmedium kann nach Abtrennung des Endproduktes durch Filtration in einem Folgeansatz wieder verwendet werden.

EP 0 054 228 A1

Croydon Printing Company Ltd.

HOECHST AKTIENGESELLSCHAFT HOE 80/F 282 Dr.ST/sch

Verfahren zur Herstellung von 1-Amino-2- oder -4-nitro-alkoxybenzolen

Die Erfindung liegt auf dem technischen Gebiet der als Zwischenprodukte verwendbaren Aniline.

Aus BIOS Final Report 986, Seite 287, ist bekannt, 1-Amino-2-nitro-4-methoxybenzol technisch durch alkalische Hydrolyse von 1-Acetylamino-2-nitro-4-methoxybenzol durch Erhitzen in verdünnter, wäßriger Natriumhydroxidlösung auf 75 bis 77°C herzustellen. Das so erhaltene 1-Amino-2-nitro-4-methoxybenzol besitzt jedoch nicht die Reinheit, die zu dessen Weiterverwendung in der Eisfarbentechnik erforderlich ist; es muß daher noch einer aufwendigen Reinigung unterworfen werden (loc. cit., S. 287 unten und S. 288 bis 290). Ebenso ist in BIOS Final Report 986 auf S. 280 und 282 eine saure Hydrolyse von 1-Acetylamino-4-nitro-2-methoxybenzol zu 1-Amino-4-nitro-2-methoxybenzol beschrieben; zur Reinigung ist aber eine wäßrige Umlösung unter Druck erforderlich. Die Hydrolyse mit der anschließenden Reinigung führt somit jeweils über zwei Stufen und erfordert große Reaktionsgefäße und erhebliche Wassermengen, wodurch sie zusätzlich mit einer starken Abwasserbelastung verbunden sind.

Reines 1-Amino-2-nitro-4-äthoxybenzol wird auf dieselbe Weise wie 1-Amino-2-nitro-4-methoxybenzol hergestellt. Dieses kann beispielsweise als Vorprodukt für die Herstellung eines Küpenfarbstoffes dienen (s. Colour Index, Third Edition, 1971, C.I.-Nr. 71120); 1-Amino-2-nitro-4-methoxybenzol kann als Diazokomponente bei der Erzeugung von wasserunlöslichen Azofarbstoffen auf der Faser nach den Methoden der Eisfarbentechnik verwendet werden (s. C.I.-Nr. 37135).

BAD ORIGINAL

Weiterhin ist aus Organic Syntheses, Coll. Vol. 3, S. 661 - 663, bekannt, 1-Acetylamino-2-nitro-4-methoxyanilin mit methanolisch-wäßriger Kalilauge zu hydrolysieren; hierbei entsteht eine dicke Paste. Zur Gewinnung der Anilinverbindung muß diese Paste mit heißem Wasser versetzt und verrührt und die Anilinverbindung durch Wasserdampf abgetrennt werden. Es ist offensichtlich, daß solch ein Verfahren sich technisch verbietet.

In Bull. Chem. Soc. Japan 10, 429 (1935) wird die gleiche Acetylaminoverbindung in die Aminoverbindung mittels alkoholischer Kaliumcarbonat-Lösung übergeführt. Die Aufarbeitung des Endproduktes erfolgt durch Konzentrieren der Reaktionslösung und nachfolgende Zugabe von Wasser, um das Produkt kristallin abzuscheiden. Auch bei dieser Verfahrensweise entsteht Abwasser, das aufgearbeitet werden muß und nicht in einen nochmaligen Reaktionsansatz zurückgeführt werden kann.

In der US-PS 2 325 797 wird die Herstellung von 1-Amino-2-nitro-4-methoxybenzol durch Hydrolyse ihrer 1-Acetylamino-Verbindung beschrieben, indem man eine Suspension dieser Acetylaminoverbindung in siedendem Alkohol langsam mit wäßriger Natronlauge versetzt. Nach Beendigung der Hydrolyse wird das Reaktionsgemisch auf Eis gegeben und mit kaltem Wasser von Alkali frei gewaschen. Auch dieses Verfahren führt zu einer erheblichen Menge an Abwasser, das in einen erneuten Ansatz nicht zurückgeführt werden kann und aufgearbeitet werden muß.

Die Nachteile der bekannten, insbesondere bisher technisch ausgeführten Verfahrensweisen der Herstellung von 1-Amino-2-nitro- bzw. -4-nitro-alkoxybenzolen aus den entsprechenden Acetylaminoverbindungen wurden nunmehr durch die vorliegende Erfindung beseitigt. Es wurde gefunden, daß man 1-Amino-2-nitro- und 1-Amino-4-nitro-alkoxybenzole auf einfachere Weise und direkt in reiner Form erhält, wenn man

BAD ORIGINAL

eine 1-Acetylamino-2-nitro-alkoxybenzol-Verbindung bzw. 1-Acetylamino-4-nitro-alkoxybenzol-Verbindung einer Alkoholyse in einem wasserfreien oder praktisch wasserfreien Alkanol von 1 bis 5 C-Atomen in Gegenwart von katalytischen Mengen eines Alkalihydroxids unterwirft.

Die so erhältliche 1-Amino-2-nitro- bzw. -4-nitro-alkoxybenzol-Verbindung besitzt eine hohe Reinheit und kann direkt zur Erzeugung von wasserunlöslichen Azofarbstoffen auf der Faser verwendet werden. Sie ist frei von isomeren Nitroverbindungen, die sich bei der Herstellung der Ausgangs-Nitroverbindungen in der Nitrierungsreaktion bilden und somit in den technischen Ausgangsprodukten bereits als Verunreinigungen enthalten sind. Im Reaktionsansatz bleiben diese isomeren Verbindungen nach der Alkoholyse gelöst zurück, während die erwünschte Aminobenzol-Verbindung rein ausfällt und, bspw. durch Filtration, abgetrennt werden kann. Eine zusätzliche Umkristallisation ist nicht erforderlich.

Das erfindungsgemäße Verfahren führt zudem zu keinem Abwasser; denn die alkoholische Reaktionslösung, die bei der Isolierung des gewünschten, reinen Produktes nicht mit Wasser versetzt wird, kann direkt wieder in einen erneuten Alkoholyseansatz als Reaktionsmedium eingesetzt werden. Durch die erfindungsgemäße Verfahrensweise gelingt es, solch eine alkoholische Reaktionslösung etwa 25 Mal als Reaktionsmedium nachfolgenden Alkoholysereaktionen der 1-Acetylamino-Ausgangsverbindungen zuzuführen. Nach Erschöpfung dieser Reaktionslösung durch Anreicherung von in ihr gelösten Nebenprodukten oder Verunreinigungen kann diese durch Verbrennen, d.h. ohne jegliche Abwasserbelastung, beseitigt werden.

Die in den Ausgangsverbindungen und Endprodukten enthaltene(n) Alkoxygruppe(n) ist (sind) vorzugsweise Alkoxygruppen von 1 bis 4 C-Atomen, insbesondere aber die Methoxygruppe oder die Äthoxygruppe. Die erfindungsgemäß als Aus-

BAD ORIGINAL

gangsverbindungen einsetzbaren Acetylamino-nitro-Verbindungen bzw. die erfindungsgemäß herstellbaren Amino-nitro-Verbindungen enthalten eine oder zwei Alkoxygruppen als Alkoxysubstituenten. Bevorzugt sind als Ausgangs- bzw. Endprodukte die Verbindungen entsprechend den Formeln (1), (2) oder (3)

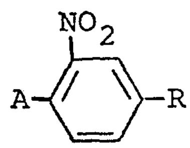
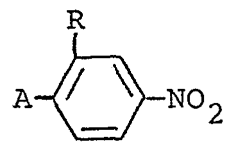
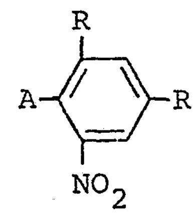

(1) (2) (3)

in welchen A für die Acetylamino- bzw. Aminogruppe steht und R eine Alkoxygruppe, vorzugsweise von 1 bis 4 C-Atomen, wie die Äthoxygruppe und insbesondere die Methoxygruppe, darstellt.

Das erfindungsgemäße Verfahren wird zweckmäßig in der Weise durchgeführt, daß man die 1-Acetylamino-2-nitro- bzw. -4-nitro-alkoxybenzol-Verbindung und das Alkalihydroxid, wie Natrium- oder Kaliumhydroxid, bevorzugt Natriumhydroxid, in einem Alkanol von 1 bis 5 C-Atomen einträgt, gegebenenfalls Aktivkohle oder ein Filtrierhilfsmittel, wie zum Beispiel Kieselgur, zugibt und das Reaktionsgemisch anschließend etwa 1,5 bis 2 Stunden unter Rückfluß rührt, dieses sodann heiß filtriert und das Filtrat abkühlen läßt, wobei das gebildete 1-Amino-2-nitro- bzw. -4-nitro-alkoxybenzol auskristallisiert und isoliert werden kann. Das Filtrat kann anschließend einem weiteren Reaktionsansatz als Reaktionsmedium dienen; erforderlichenfalls muß nur ein geringer Verlust an Alkanol ergänzt werden.

Das als Katalysator verwendete Alkalihydroxid wird mit einer Menge bis zu 10 Gew.-%, beispielsweise von 0,5 bis 10 Gew.-%, insbesondere etwa 1 bis 5 Gew.-%, berechnet auf die Acetylamino-Ausgangsverbindung, in den Reaktionsansatz eingesetzt.

Die Alkoholyse wird bevorzugt bei einer Temperatur zwischen

BAD ORIGINAL

0054228

60 und 100°C durchgeführt. Insbesondere führt man sie unter Rückfluß des Lösemittels (Alkanols) bzw. des während der Umsetzung entstehenden Alkanol/Essigsäurealkanolester-Gemisches aus. Das als Lösemittel bei der Alkoholyse verwendete Alkanol ist beispielsweise n-Propanol oder Isopropanol, insbesondere Methanol oder Äthanol; es soll wasserfrei oder praktisch wasserfrei sein, um eine hohe Ausbeute zu gewährleisten. Der Wassergehalt der technischen Alkanole, die immer einen geringen Wasseranteil besitzen, sollte deshalb nicht höher als 2 Gew.-% betragen.

Gegenüber den bekannten Verfahren sind bei der Ausführung des erfindungsgemäßen Verfahrens weniger Verfahrensschritte erforderlich, die mit einem geringeren apparativen Aufwand verbunden sind. Die etwa 25-malige Wiederverwendungsmöglichkeit des Alkohols (des Reaktionsmediums) in weiteren Ansätzen, eine Verfahrensmaßnahme, die mit keiner Abwasserbelastung verbunden ist, hat zudem eine erhebliche Bedeutung in der technischen Ausführungsmöglichkeit, die dem erfindungsgemäßen Verfahren einen erheblichen technischen Fortschritt zusichert.

Die nachstehenden Beispiele dienen zur Erläuterung der Erfindung. Die Teile sind Gewichtsteile.

Beispiel 1

In 1108 Teile Methanol werden 184,4 Teile 1-Acetylamino-2-nitro-4-methoxybenzol, 2 Teile Natriumhydroxid, 6 Teile Entfärbekohle und 2 Teile eines Filtrierhilfsmittels (Kieselgur) eingetragen. Der Ansatz wird zum Sieden erhitzt, etwa 90 Minuten unter Rückfluß gerührt und heiß filtriert. Der Filterrückstand wird mit 79 Teilen Methanol von 50 bis 60°C gewaschen. Das Filtrat läßt man innerhalb von 2 Stunden auf 0 bis 5°C abkühlen und rührt es noch eine Stunde bei dieser Temperatur nach.

Das abgeschiedene 1-Amino-2-nitro-4-methoxybenzol wird abgesaugt, mit etwa 300 Teilen kaltem Wasser gewaschen und getrocknet. Man erhält 133,5 Teile dieser Verbindung mit einem Reingehalt von 99,6 %. Der Schmelzpunkt beträgt 122 bis 124°C, und die Ausbeute entspricht 90,5 % der Theorie.

Das Produkt kann direkt als Diazokomponente ("Echtfärbebase") in der Eisfarbentechnik zur Herstellung von wasserunlöslichen Azofarbstoffen auf der Faser eingesetzt werden.

Beispiel 2

Man verfährt in der in Beispiel 1 angegebenen Verfahrensweise, setzt jedoch anstelle von 1-Acetylamino-2-nitro-4-methoxybenzol als Ausgangsverbindung die äquivalente Menge 1-Acetylamino-2-nitro-4-äthoxybenzol ein. Man erhält das 1-Amino-2-nitro-4-äthoxybenzol in 89,7 %iger Ausbeute mit einem Reingehalt von 98 % und einem Schmelzpunkt von 111 bis 113°C.

Das Produkt kann direkt in die Reduktionsreaktion zur Herstellung von 1,2-Diamino-4-äthoxybenzol als Zwischenprodukt für die Herstellung eines Küpenfarbstoffes eingesetzt werden.

BAD ORIGINAL

Beispiel 3

In 600 Teile Äthanol werden 99 Teile 1-Acetylamino-2-nitro-4,6-dimethoxy-benzol, 1 Teil Natriumhydroxid, 4 Teile Entfärbekohle und 2 Teile eines Filterhilfsmittels eingetragen. Man erhitzt auf Rückflußtemperatur (etwa 80 bis 85°C) und rührt zwei Stunden bei Rückfluß weiter. Anschließend wird der Ansatz heiß filtriert, das Filtrat unter Rühren auf 0 bis 5°C abgekühlt und das ausgefallene 1-Amino-2-nitro-4,6-dimethoxy-benzol abgesaugt, mit 200 Teilen kaltem Wasser gewaschen (das nicht in das äthanolische Filtrat gelangen soll) und getrocknet. Man erhält 80,0 Teile dieser Verbindung mit einem Reingehalt von 99,7 % entsprechend 97,9 % der Theorie bei einem Schmelzpunkt von 112 - 113°C.

Beispiel 4

Man verfährt in der im Beispiel 3 angegebenen Verfahrensweise, setzt jedoch anstelle von 1-Acetylamino-2-nitro-4,6-dimethoxy-benzol die äquivalente Menge 1-Acetylamino-4-nitro-2-methoxybenzol ein. Das gewonnene 1-Amino-4-nitro-2-methoxy-benzol wird in einer Ausbeute von 91,8 % mit einem Reingehalt von 98,7 % und einem Schmelzpunkt von 145 bis 146°C erhalten.

Patentansprüche:

1. Verfahren zur Herstellung von 1-Amino-2-nitro-alkoxybenzolen oder 1-Amino-4-nitro-alkoxybenzolen aus 1-Acetylamino-2-nitro-alkoxybenzolen bzw. 1-Acetylamino-2-nitro-alkoxybenzolen, dadurch gekennzeichnet, daß man ein 1-Acetylamino-2-nitro- bzw. -4-nitro-alkoxybenzol einer Alkoholyse in einem wasserfreien oder praktisch wasserfreien Alkanol von 1 bis 5 C-Atomen in Gegenwart von katalytischen Mengen eines Alkalihydroxids unterwirft.

2. Verfahren nach Anspruch 1, dadurch gekennzeichnet, daß das Acetylamino-nitro-alkoxybenzol eine Verbindung der allgemeinen Formel

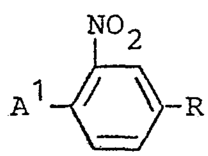

ist, in welcher $A^1$ für die Acetylaminogruppe steht und R eine Alkoxygruppe bedeutet, und diese zur Aminoverbindung der allgemeinen Formel

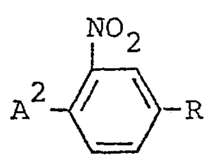

in welcher $A^2$ die Aminogruppe ist und R die obengenannte Bedeutung besitzt, hydrolysiert wird.

3. Verfahren nach Anspruch 1, dadurch gekennzeichnet, daß das Acetylamino-nitro-alkoxybenzol eine Verbindung der allgemeinen Formel

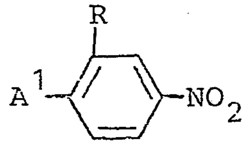

ist, in welcher $A^1$ für die Acetylaminogruppe steht und R eine Alkoxygruppe bedeutet, und diese zur Aminoverbindung der allgemeinen Formel

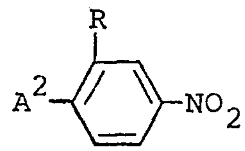

in welcher $A^2$ die Aminogruppe ist und R die obengenannte Bedeutung besitzt, hydrolysiert wird.

4. Verfahren nach Anspruch 1, dadurch gekennzeichnet, daß das Acetylamino-nitro-alkoxybenzol eine Verbindung der allgemeinen Formel

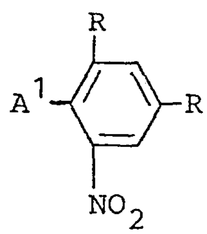

ist, in welcher $A^1$ für die Acetylaminogruppe steht und R eine Alkoxygruppe bedeutet, und diese zur Aminoverbindung der allgemeinen Formel

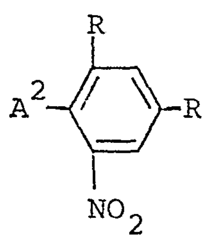

in welcher $A^2$ die Aminogruppe ist und R die obengenannte Bedeutung besitzt, hydrolysiert wird.

5. Verfahren nach einem der Ansprüche 1 bis 4, dadurch gekennzeichnet, daß man die alkalische Hydrolyse bei einer Temperatur zwischen 60 und 100°C durchführt.

6. Verfahren nach einem der Ansprüche 1 bis 5, dadurch gekennzeichnet, daß man das alkoholische Reaktionsmedium nach Isolierung des Endproduktes in einem Nachfolgeansatz wiederum als Reaktionsmedium einsetzt.

7. Verfahren nach einem der Ansprüche 1 bis 6, dadurch gekennzeichnet, daß man das Alkalihydroxid in einer Menge von 1 bis 5 Gew.-%, berechnet auf die Acetylamino-Ausgangsverbindung, einsetzt.

8. Verfahren nach einem der Ansprüche 1 bis 4 oder 7, dadurch gekennzeichnet, daß man die Hydrolyse bei Rückflußtemperatur des Reaktionsmediums durchführt.

9. Verfahren nach Anspruch 1, 5, 6, 7 oder 8, dadurch gekennzeichnet, daß das 1-Acetylamino-2-nitro-4-alkoxybenzol das 1-Acetylamino-2-nitro-4-methoxybenzol oder das 1-Acetylamino-2-nitro-4-äthoxybenzol ist.

0054228

Europäisches Patentamt

# EUROPÄISCHER RECHERCHENBERICHT

Nummer der Anmeldung
EP 81 11 0103

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | REC. TRAV. CHIMIQUES PAYS-BAS, Band 62, 1943, Seiten 201-204 Den Haag, NL. P.E. VERKADE et al.: "Deacylation of acetylamino compounds containing a nitro group in the ortho or para position" * Seiten 203-204: a,d * | 1-5,7-9 |
| | -- | |
| X | REC. TRAV. CHIMIQUES PAYS-BAS, Band 70, 1951, Seiten 340-342 Den Haag, NL. H.J.B. BIEKART et al.: "Preparation and ultraviolet absorption spectrum of meta-nitroisopropylbenzene" * Seite 342: Zeilen 7-17 * | 1-9 |
| | -- | |
| X | GB - A - 597 835 (N.V. POLAK & SCHWARZ'S ESSENCEFABRIEKEN) * Seite 2, Zeilen 11-61; Seite 2, Zeilen 83-89, 102-112 * | 1-5,7-9 |
| | ------ | |

KLASSIFIKATION DER ANMELDUNG (Int Cl 3)

C 07 C 93/14

RECHERCHIERTE SACHGEBIETE (Int Cl 3)

C 07 C 93/00

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

λ Der vorliegende Recherchenbericht wurde fur alle Patentansprüche erstellt.

| Recherchenort | Abschlußdatum der Recherche | Prüfer |
|---|---|---|
| Den Haag | 10-03-1982 | PAUWELS |